# EUROPEAN PATENT APPLICATION

(11) **EP 1 569 000 A2**
(43) Date of publication of application: **31.08.2005**
(21) Application number: 05250976.7
(22) Date of filing: 21.02.2005
(51) Int. Cl.: G01R 1/073

(54) **Probe card**

(30) Priority: 24.02.2004 JP 2004047657
(71) Applicant: NIHON DENSHIZAIRYO KABUSHIKI KAISHA, Hyogo (JP)
(72) Inventor: Kimura, Teppei, Kobe-shi Hyogo, 651 2413 (JP)
(74) Representative: Beresford, Keith Denis Lewis

(57) **Abstract**

A probe card is capable of readily arranging a wiring pattern without forming the through hole while using a silicon substrate as a supporting substrate.

The probe card comprises: a supporting substrate 100, one surface of which is formed in a pyramid shape having one step; a plurality of probes 200 arranged on the surface of a thick part of the supporting substrate 100; a plurality of bumps 110 arranged on the surface of a thin part of the supporting substrate 100; and a plurality of wiring patterns 120 which are arranged on the one surface of the supporting substrate 100 and electrically connect each of the probes 200 with each of the bumps 110.

## Description

The present invention relates to a probe card for use in measurement of electrical characteristics of an object to be measured.

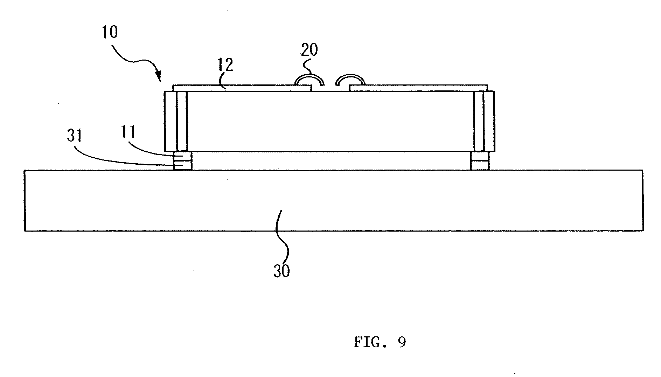

Probe cards of this kind include one as shown in Fig. 9, which comprises: a supporting substrate 10 having through holes 11 filled with a conductive material; probes 20 which are arranged on the surface of this supporting substrate 10 and also electrically connected with the conductive material in the through holes 11 via wiring patterns 12; and a main substrate 30 which is arranged opposite to the rear surface of the supporting substrate 10, and on the surface of which, opposed to the rear surface of the supporting substrate, bumps 13 are arranged, wherein bumps 13 to be electrically connected with the conductive material in the through holes 11 are arranged on the rear surface of the supporting substrate 10, and the bumps 13 are contactable with the bumps 13 arranged on the main substrate 30.

Since this probe card is used in a broad range of temperatures in measurement, for matching with thermal expansion of an object to be measured, a silicon substrate, which thermally expands along with the object to be measured, has been used as the supporting substrate, as shown, for example, in Japanese Patent Laid-Open No. 2000-121673.

However, for using a silicon substrate as the supporting substrate, it needs to have a thickness not smaller than 500 µm in consideration of curling or solidity of the silicon substrate. The use of the silicon substrate having a thickness not smaller than 500 µm makes it extremely difficult to form the through hole. This is because, in formation of the through hole where a hole not larger than φ100 µm is formed to be filed with the conductive material, when the silicon substrate having a thickness not smaller than 500 µm is used, the hole can be formed but cannot be well-filled with the conductive material, or even if the hole can be well-filled, it takes a great amount of time, raising an essential problem of cost increase.

This problem seems solvable by not forming the through hole in the supporting substrate of the probe card but arranging the bump on the surface of the supporting substrate to be connected with a probe via a wiring pattern. However, there are some cases where the height of the bump becomes higher than the height of the probe having been made finer corresponding to increased integration of the object to be measured. Furthermore, since the bump is connected with a flexible substrate or the like so as to be electrically connected with the bump of the main substrate, the height of the bump is added with the height of the flexible substrate to become even higher than the height of the probe.

With the bump higher than the probe, as thus described, in using the probe card for measurement of the object to be measured, the bump comes into contact with an electrode or the surface of the object to be measured earlier than the probe, and thereby the probe card can no longer be used in measurement of a highly integrated object to be measured. Namely, there is another problem in that the probe card cannot correspond to the forgoing object to be measured. While using a bump having a smaller height than that of the probe may be considered, since the probe is elastically transformed in measurement so as to obtain a prescribed contact pressure with the electrode of the object to be measured, it is still possible for the bump to come into contact with the object to be measured. That is, the above problem still remains.

The present invention was made in view of the above circumstances, and has a first object to provide a probe card capable of corresponding to a highly integrated object to be measured without forming the forgoing through hole while using a silicon substrate as the supporting substrate. The present invention has a second object to provide a probe card capable of readily forming the forgoing through hole therein, while using a silicon substrate as the supporting substrate.

According to a first aspect of the present invention, a first probe card comprises: a supporting substrate, one surface thereof provided with a plurality of probes and a plurality of bumps electrically connected with the probes via wiring patterns; and a main substrate, opposed to the other surface of the supporting substrate and electrically connected with the bumps on the supporting substrate, characterized in that the one surface of said supporting substrate is formed in a convex shape having one or more steps, and said probes are arranged on a surface of the top step in the one surface of the supporting substrate, while said bumps are arranged on a surface of a lower step in the one surface of the supporting substrate.

According to a second aspect of the present invention, a probe card comprises: a supporting substrate, one surface thereof provided with a plurality of probes and the other surface thereof provided with a plurality of bumps electrically connected with said probes; and a main substrate, opposed to the other surface of the supporting substrate and electrically connected with the bumps on the supporting substrate, characterized in that the one surface of said supporting substrate is formed in a convex shape having one or more steps, a plurality of wiring patterns are arranged from a surface of a top step through a surface of a bottom step in the one surface of the supporting substrate, said probes each electrically connected with one end of each of said wiring patterns are arranged on the surface of the top step while a plurality of first through holes are formed in the bottom step in the one surface of the supporting substrate, and each of said bumps is electrically connected with the other end of each of said wiring patterns via a conductive material arranged within each of the first through holes.

In the probe card according to the first aspect of the invention, the bumps are arranged at a larger pitch spacing than the probes. Similarly, in the probe card according to the second aspect of the invention, the first through holes are arranged at a larger pitch spacing than the probes.

A ground layer and an insulating layer are sequentially stacked on the one surface of the supporting substrate, and the wiring patterns are formed on the surface of the insulating layer.

Further, second through holes are formed from the surface of the top step of the one surface of the supporting substrate through the other surface of the supporting substrate. In this case, on the other surface of the supporting substrate, a concave part is preferably arranged at a place with the second through holes formed therein.

The one surface of the supporting substrate is preferably formed in a pyramid shape having one or more steps.

In the case of the probe card according to claim 1 of the present invention, the one surface of the supporting substrate is formed in a convex shape having one or more steps, and the probes are arranged on the surface of the top step while the bumps are arranged on the surface of either of the lower steps. This prevents the bumps from coming into contact with the electrode or the surface of the object to be measured even when the probes are brought into contact with the electrode of the object to be measured. Therefore, even if the integration of the object to be measured is increased and the probes are made finer associated with the increased integration, the bumps are not arranged in a higher position than the probe, thereby enabling sufficient correspondence to the object to be measured. Furthermore, the one surface of the supporting substrate is formed in a convex shape having one or more steps. This figuration can secure necessary thickness of the supporting substrate.

In the case of the probe card according to claim 2 of the present invention, the one surface of the supporting substrate is formed in a convex shape having one or more steps. This figuration can secure necessary thickness of the supporting substrate. Furthermore, since a thin part (from the surface of the bottom step of the one surface of the supporting substrate through the another surface of the supporting substrate) is arranged by forming the one surface of the supporting substrate into a convex shape having one or more steps, it is possible to readily form the first through holes by formation of the first through holes to be filled with a conductive material in the thin part. Therefore, the process time for opening the first through holes can be reduced as compared to conventional examples, which can promote cost reduction. Furthermore, such formation of the first through holes is advantageous in facilitating arrangement of the wiring patterns since the wiring patterns can also be arranged on the rear surface of the supporting substrate through the first through holes.

In the case of the probe card according to claim 3 of the present invention, the bumps or the first through holes are arranged at a wider pitch spacing than the probes. This can facilitate arrangement of the wiring patterns.

In the case of the probe card according to claim 4 of the present invention, the second through holes are formed in a thick part of the supporting substrate (from the surface of the top step of the one surface of the supporting substrate through the another surface of the supporting substrate), and each of probes for connection are to be inserted into each of the second through holes. Since the probes for connection can be used for power source lines or signal source lines, the number of wiring patterns to be arranged on the surface of the supporting substrate can be reduced as compared to conventional examples. This can facilitate arrangement of the wiring patterns.

In the case of the probe card according to claim 5 of the present invention, a concave part is arranged at a place, with the second through holes formed therein, in the other surface of the supporting substrate. Hence, the formation of the second through holes in the arranged concave part can facilitate arrangement of the second through holes as compared with the case where the second through holes are formed in the thick part of the supporting substrate.

In the case of the probe card according to claim 6 of the present invention, the same effect is exerted as in claim 1.

A number of embodiments of the present invention will now be described, by way of example only, with reference to the accompanying drawings in which:
Fig. 1 is a schematic sectional view of a probe card according to a first embodiment of the present invention;
Fig. 2 is a schematic plan view of a supporting substrate of the same probe card;
Fig. 3 is a schematic expanded view of the α part in Fig. 1;
Fig. 4 is a view showing an example of changing the design of the same probe card, where (a) is a schematic plan view of the supporting substrate, and (b) is a sectional view taken along Line A-A of (a);
Fig. 5 is a schematic sectional view of the supporting substrate showing another example of changing the design of the same probe card;
Fig. 6 is a schematic sectional view of a probe card according to a second embodiment of the present invention;
Fig. 7 is a view showing an example of changing the design of the same probe card, where (a) is a schematic plan view of the supporting substrate, and (b) is a sectional view taken along Line A-A of (a);
Fig. 8 is a schematic sectional view of the supporting substrate showing another example of changing the design of the same probe card; and
Fig. 9 is a schematic sectional view of a probe card of a conventional example, as already described.

### First Embodiment

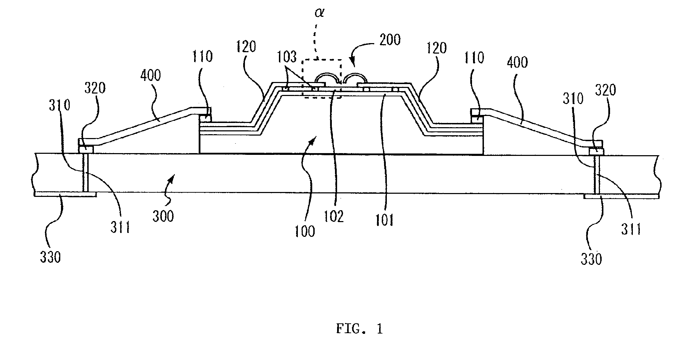

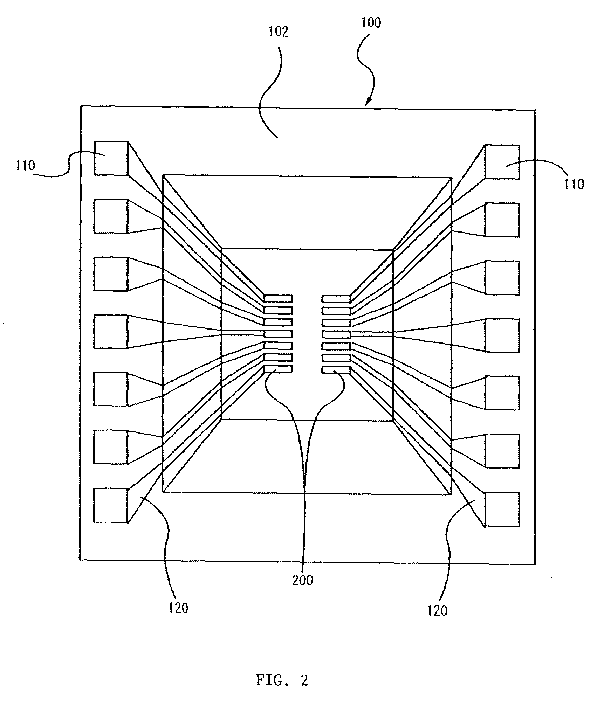

The probe card A shown in Fig. 1 and Fig. 2 is a sensing part of a measuring device (not shown) for measuring an object to be measured (not shown), and comprises: a supporting substrate 100; probes 200 to be arranged on one surface of the supporting substrate 100; and a main substrate 300 disposed opposite to the other surface of the supporting substrate 100. Each of the parts is described in detail below.

A silicon substrate is used for the supporting substrate 100. The one surface of the supporting substrate 100 has been formed into a pyramid shape having one step (i.e. convex shape) by anisotropic etching. This step part of the one surface of the supporting substrate 100 is sloped. Further, on the one surface of the supporting substrate 100, a grand layer 101, an insulating layer 102 and wiring patterns 120 are sequentially stacked to be formed by vapor deposition, plating, etching or the like. The thick part of the supporting substrate 100 has a thickness of 500 to 1500 µm while the thin part thereof is 100 to 500 µm.

On the surface of the thick part of the supporting substrate 100 (namely, on the one ends of the wiring patterns 120 on the surface of the top step in the one surface of the supporting substrate 100), a plurality of probes 200 are arranged in two rows at a prescribed pitch spacing. The probes 200 in the respective rows are opposed to each other. On the other hand, on each edge on the surface of the thin part of the supporting substrate 100 (namely, on the other ends of the wiring patterns 120 on the surface of each of the lower steps in the one surface of the supporting substrate 100), a plurality of bumps 110 are arranged at a wider pitch spacing than the probes 200. The probes 200 and the bumps 110 are electrically connected with each other via the plurality of wiring patterns 120. A ground layer 101, an insulating layer 102 and the wiring patterns 120 are sequentially stacked on the one surface of the supporting substrate 100, to constitute microstrip lines.

Further, the insulating layer 102 on the supporting substrate 100 is provided with a circuit element 103 electrically connected with the wiring patterns 120. This circuit element 103 is an element necessary in electrical measurement using the probes 200, and herein, a capacitor, which functions as a so-called "passcon" (bypass capacitor), and the circuit element, which functions as BOST (Build Out Self Test) for aiding a test (namely, measurement of electric characteristics of an object B to be measured), are used. The capacitor serves to promote improvement in high frequency characteristics. The circuit element, having the function as BOST, serves in a varying manner depending on the contents of the test on the object B to be measured.

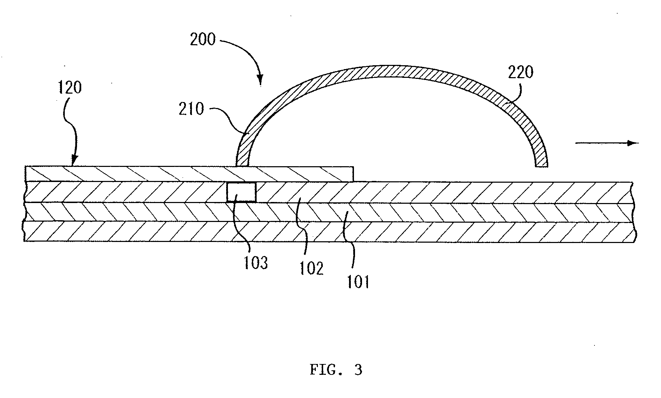

A light-sensitive film is applied to the wiring patterns 120 on the thick part of the supporting substrate 100, a pattern is formed on this light-sensitive film to be plated, which is repeated to integrally form the probes 200 on the wiring patterns 120. As shown in Fig. 3, each of the probes 200 is formed to have a first one-quarter arc part 210 with the one end thereof supported by the insulating layer 102, and a second one-quarter arc 220 connected with the other end of the first one-quarter arc part 210, and made slightly shorter than the first one-quarter arc part 210. The tops of the probes 200 are contact parts to come into contact with the electrode of the object to be measured.

The main substrate 300 is a substrate to be mounted in a state of being in contact with the supporting substrate 100, and a printed circuit board is used here. A plurality of through holes 310 filled with a conductive material 311 are formed in the main substrate 300. Further, on the one surface of the main substrate 300 (the surface on which the supporting substrate 100 is mounted) arranged are a plurality of bumps 320 to be electrically connected with the conductive material 311 in the through holes 310. The bumps 320 are connected with the bumps 110 of the supporting substrate 100 via a flexible substrate 400.

On the other surface of the main substrate 300, one end of each of wiring patterns 330 to be electrically connected with the conductive material 311 in the through holes 310 are formed. The other ends of the wiring patterns 330 are electrically connected with an outer electrode which is not shown, and the wiring patterns 330 are electrically connected to the measuring device through this outer electrode.

Namely, a signal outputted from the measuring device is transmitted through the outer electrode, the wiring patterns 330, the conductive material 311, the bumps 320, the flexible substrate 400, the bumps 110, the wiring patterns 120, and the probes 200, sequentially.

The main substrate 300 of as thus constituted is mounted on a prober of the measuring device, and the probe card A is then used for measurement of electrical characteristics of the object to be measured. A method of use thereof is specifically described below.

First, a probe-driving device is activated, and the supporting substrate 100 and the object to be measured are made relatively close to each other. This brings the tops of the probes 200 into contact with the electrode of the object to be measured. Subsequently, the supporting substrate 100 and the object to be measured are further made relatively close to each other so that the probes 200 are pressed by the electrode of the object to be measured (namely, overdrive is executed). At this time, since the probes 200 are arranged on the thick part of the supporting substrate 100, the supporting substrate 100 is not warped under the pressing force acted through the probes 200.

In this process, as the probes 200 are elastically transformed, the top of the second one-quarter arc 220 in each of the probes 200 comes into contact with the surface of the supporting substrate 100, as shown in Fig. 3, and then moves (in the arrow direction of Fig.3) on the surface of the supporting substrate 100. Simultaneously, the tops of the probes 200 slide on the electrode of the object to be measured. It is thereby possible to secure the prescribed contact pressure necessary for conducting electricity between the probes 200 and the electrode of the object to be measured, as well as a prescribed amount scrubbed, so that stable contact can be promoted.

In the case of the probe card A as thus described, since the probes 200 are arranged on the thick part of the supporting substrate 100, the supporting substrate 100 is not warped under the pressing force that acts through the probes 200 when the probes 200 and the electrode of the object to be measured are brought into contact with each other. This enables stable measurement. Further, since the one surface of the supporting substrate 100 is formed in a pyramid shape having one step and the probes 200 are arranged on the surface of the thick part (namely, on the surface of the top step) while the bumps 110 are arranged on the surface of the thin part (namely, on the surface of the lower steps), when the probes 200 comes into contact with the electrode of the object to be measured, the bumps 110 are not brought into contact with the object to be measured. Namely, even if the integration of the object to be measured is increased and the probes 200 are made finer associated with the increased integration, the bumps 110 are not arranged in a higher position than the probes 200, thereby enabling sufficient correspondence to the object to be measured.

Although it was described above that the one surface of the supporting substrate 100 is formed in a pyramid shape having one step, it may be formed in a convex shape having one or more steps, and may for example be formed in a staircase shape. Although it was described that the ground layer 101 and the insulating layer 102 are sequentially stacked on the supporting substrate 100, those layers are not necessarily arranged. In the case of not arranging them, the bumps 110, the wiring patterns 120 and the probes 200 are formed on the one surface of the supporting substrate 100. Further, in the case of forming the one surface of the supporting substrate 100 into a convex shape having one or more steps, the bumps 110 may be arranged on the surface of any of lower steps. Moreover, although it was described that the supporting substrate 100 is a silicon substrate, it is possible, naturally, to use another substrate.

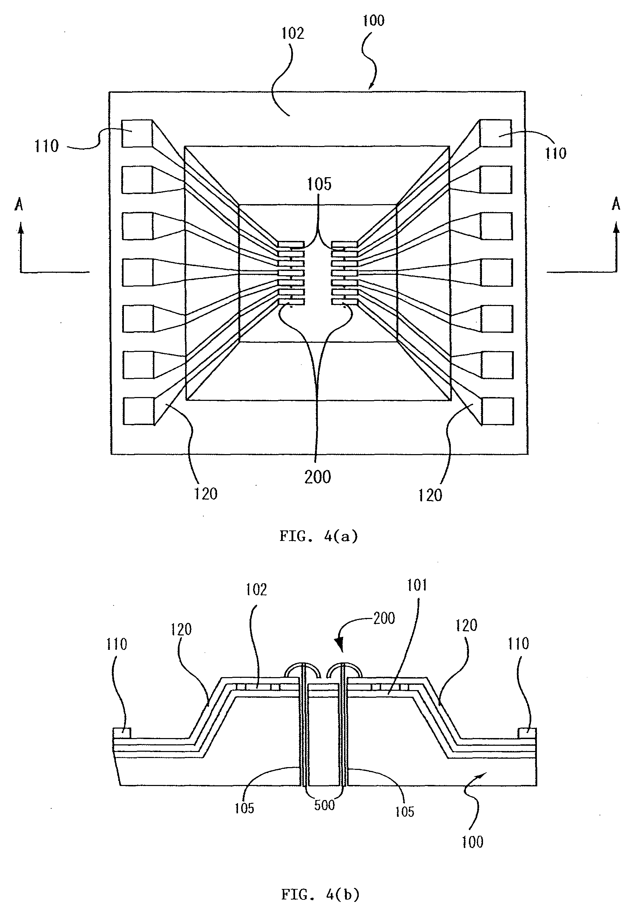

Further, as shown in Fig. 4, through holes 105 with φ50 to 100 µm (namely, second through holes) can be formed in the thick part of the supporting substrate 100. The through holes 105 are formed from the surface of the top step in the one surface of the supporting substrate 100 through the other surface of the supporting substrate 100. Moreover, each of probes 500 for connection is inserted into each of the through holes 105. In the case of using the probes 500 for connection as power source lines, each of the through holes 105 is arranged between the probes 200, as shown in Fig. 4(b). Namely, the front end of each of the probes 500 for connection protrudes from between the probes 200 to come into contact with the electrode for power source of the object to be measured while the back end thereof is electrically connected with the measuring device.

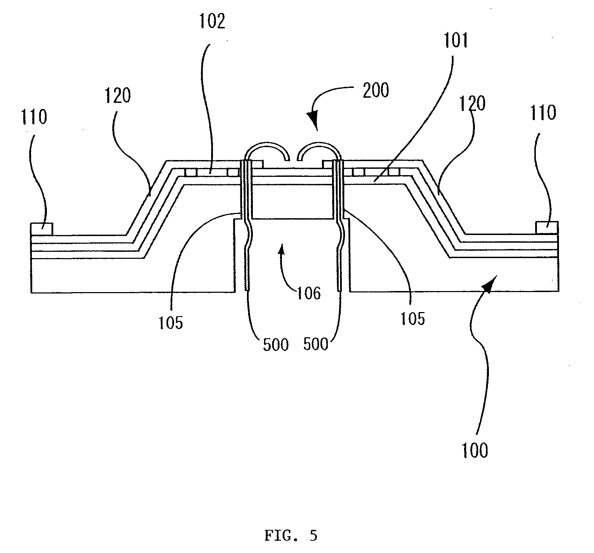

On the other hand, in the case of using the probes 500 for connection as signal lines, the through holes 105 are formed in such a position that the probes 500 for connection are electrically connectable with the wiring patterns 120. While being in contact with the wiring patterns 120, the probes 500 for connection come into contact with wiring patterns (not shown) on the one surface of the main substrate 300, to electrically connect the main substrate 300 with the wiring patterns 120. Further, as shown in Fig. 5, a cylindrical concave part 106 may be arranged in the other surface of the thick part of the supporting substrate 100, followed by formation of the through holes 105 from the concave part 106. This arrangement can facilitate formation of the through holes 105. It is to be noted that the probes 500 for connection are configured to be springy so as to come into contact with the electrode of the object to be measured, the wiring patterns 120 or the wiring patterns on the main substrate 300, by prescribed contact pressure. For example, the probes 500 can include springs or the like therebetween, or be provided with a bent part (cf. Fig. 5).

The shape of the probes 200 to be used is not limited to the forgoing shape of the example, and may be any shape.

### Second Embodiment

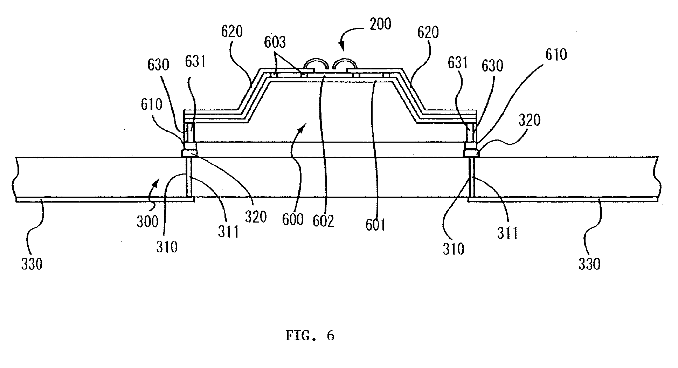

A probe card A' shown in Fig. 6 comprises a supporting substrate 600 and a plurality of probes 200 arranged on the supporting substrate 600. This probe card A' and the probe card A have almost the same configuration, but are different in that a plurality of through holes 630 (first through holes) are formed in the thin part of the supporting substrate 600. In the following, this difference is described in detail whereas explanations of the overlapped parts are omitted.

The supporting substrate 600 has almost the same configuration as that of the supporting substrate 100, except that the through holes 630 are formed in the thin part, and that bumps 610 are arranged in such a position as can come into contact with a conductive material 631 in the through holes 630 on the other surface, not the one surface, of the supporting substrate 600. Holes with φ50 to 100 µm are formed from the surface of the lower steps in the one surface of the supporting substrate 600 through the other surface of the supporting substrate 600, which are then filled with the conductive material 631, to constitute the through holes 630.

After the formation of the through holes 630, in the same manner as in the case of the supporting substrate 100, a ground layer 601, an insulating layer 602 and the wiring patterns 620 are sequentially formed on the one surface of the supporting substrate 600. The conductive material 631 in the through holes 630 electrically connects the ground layer 601 with the bumps 610. The ground layer 601 and the wiring patterns 620 are electrically connected with each other by vapor deposition of the wiring patterns 620 on an opening formed in the insulating layer 602. With this supporting substrate 600 mounted on the main substrate 300, the bumps 610 on the supporting substrate 600 come into contact with the bumps 320 on the main substrate 300. This leads to electrical connection between the supporting substrate 600 and the main substrate 300.

Namely, a signal outputted from the measuring device is transmitted through the outer electrode (not shown) of the main substrate 300, the wiring patterns 330, the conductive material 311, the bumps 320, the bumps 610, the conductive material 631, the ground layer 601, the wiring patterns 620, and the probes 200, sequentially.

The main substrate 300 of the probe card A' as thus constituted is mounted on the prober of the measuring device, and the probe card A' is then used for measurement of electrical characteristics of the object to be measured. A using method thereof is specifically described below.

First, a probe-driving device is activated, and the supporting substrate 600 and the object to be measured are made relatively close to each other. This brings the tops of the probes 200 into contact with the electrode of the object to be measured. Subsequently, the supporting substrate 600 and the object to be measured are further made relatively close to each other so that the probes 200 are pressed by the electrode of the object to be measured (namely, overdrive is executed). At this time, since the probes 200 are arranged on the thick part of the supporting substrate 600, the supporting substrate 600 is not warped under pressing force acted through the probes 200.

In this process, as the probes 200 are elastically transformed, the top of the second one-quarter arc 220 in each of the probes 200 comes into contact with the surface of the supporting substrate 600, and then moves on the surface of the supporting substrate 600. Simultaneously, the tops of the probes 200 slide on the electrode of the object to be measured. It is thereby possible to secure prescribed contact pressure necessary for conducting electricity between the probes 200 and the electrode of the object to be measured, as well as a prescribed amount scrubbed, so that stable contact can be promoted.

In the case of the probe card A' as thus configured, the same effect as that of the probe card A is exerted. Furthermore, since a thin part is provided in the supporting substrate 600 by forming the one surface of the supporting substrate 600 into a pyramid shape having one step, it is possible to readily form the through holes 630 by forming the through holes 630 in the thin part. Namely, the process time for forming the through holes 630 is substantially reduced as compared to conventional examples, which can lower the operation cost to promote cost reduction. Moreover, in this configuration, the supporting substrate 600 and the main substrate 300 can be electrically connected with each other only by mounting the supporting substrate 600 on the main substrate 300, which is readily conducted as compared with the case like the probe card A where the flexible substrate 400 is used for electrical connection. That is, also from this aspect, the operation time can be reduced because the probe card A' can be readily assembled, thereby to promote cost reduction.

Although it was described above that the one surface of the supporting substrate 600 is formed in a pyramid shape having one step, it may be formed in a convex shape having one or more steps, and may for example be formed in a staircase shape. Although it was described that the ground layer 601 and the insulating layer 602 are sequentially stacked on this supporting substrate 600, those are not necessarily arranged. Moreover, although it was described that the supporting substrate 600 is a silicon substrate, it is possible, naturally, to use another substrate.

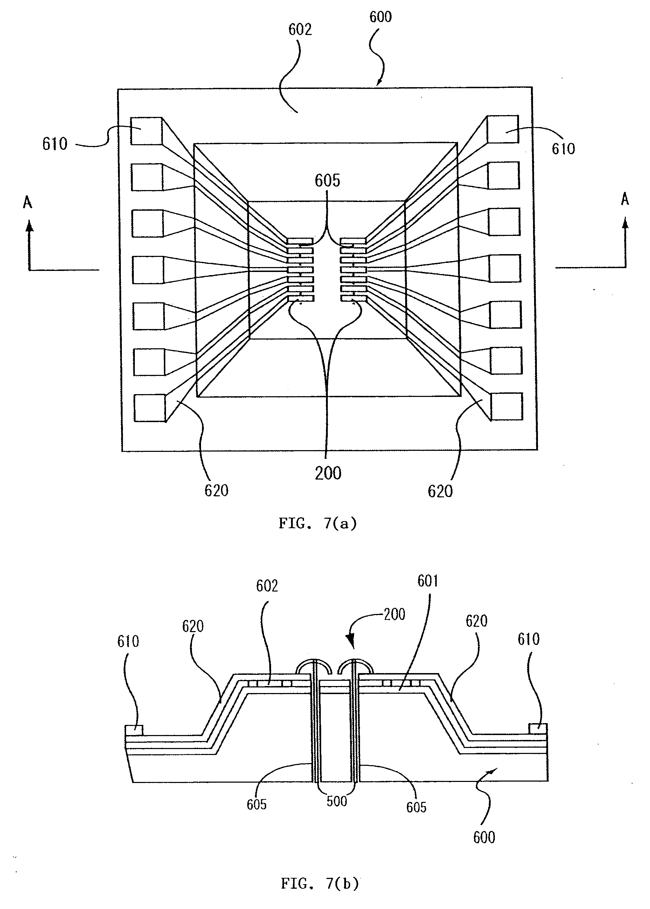

Further, as shown in Fig. 7, through holes 605 with φ50 to 100 µm (namely, second through holes) can be formed in the thick part of the supporting substrate 600. The through holes 605 are formed from the surface of the top step in the one surface of the supporting substrate 600 through the other surface of the supporting substrate 600. Moreover, each of the probes 500 for connection is inserted into each of the through holes 605. In the case of using the probes 500 for connection as power source lines, each of the through holes 605 is arranged between the probes 200. Namely, the front end of each of the probes 500 for connection protrudes from between the probes 200 to come into contact with the electrode for power source of the object to be measured while the back end thereof is electrically connected with the measuring device.

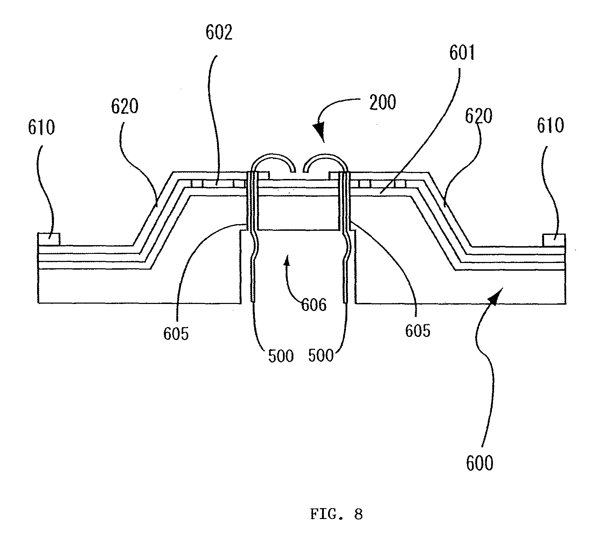

On the other hand, in the case of using the probes 500 for connection as signal lines, the through holes 605 are formed in such a position that the probes 500 for connection are electrically connectable with the wiring patterns 620. Namely, while being in contact with the wiring patterns 620, the probes 500 for connection come into contact with wiring patterns (not shown) on the one surface of the main substrate 300, to electrically connect the supporting substrate 600 with the main substrate 300. Further, as shown in Fig. 8, a cylindrical concave part 606 may be arranged in the other surface of the thick part of the supporting substrate 600, followed by formation of the through holes 605 from the concave part 606. This arrangement can facilitate formation of the through holes 605. It is to be noted that the probes 500 for connection are configured to be springy so as to come into contact with the electrode of the object to be measured, the wiring patterns 620 or the wiring patterns on the main substrate 300, by prescribed contact pressure. For example, the probes 500 can include springs or the like therebetween, or be provided with a bent part (c.f. Fig. 8).

Although it was described that the through holes 630 are configured to be filled with the conductive material 631, the conductive material 631 may be plated on the inner wall of the through holes 630 so as to electrically connect the bumps 610 with the ground layer 601 through the conductive material 631 on the inner wall.

The shape of the probes 200 to be used is not limited to the forgoing shape of the example, and may be any shape.

Although it was described that the main substrate 300 can be mounted on the supporting substrate 100 or the supporting substrate 600 in a contacting state, it is possible, naturally, that the main substrate 300 is connected with the supporting substrate 100 or the supporting substrate 600 in a separate state via a supporting member such as a threaded rod.

## Claims

1. A probe card, comprising:
a supporting substrate, one surface thereof provided with a plurality of probes and a plurality of bumps electrically connected with the probes via wiring patterns; and a main substrate, opposed to an other surface of the supporting substrate and electrically connected with the bumps on the supporting substrate, **characterized in that**
the one surface of said supporting substrate is formed in a convex shape having one or more steps, and said probes are arranged on a surface of a top step in the one surface of the supporting substrate, while said bumps are arranged on a surface of a lower step in the one surface of the supporting substrate.

2. A probe card, comprising:
a supporting substrate, one surface thereof provided with a plurality of probes and an other surface thereof provided with a plurality of bumps electrically connected with said probes; and a main substrate, opposed to the other surface of the supporting substrate and electrically connected with the bumps on the supporting substrate, **characterized in that**
the one surface of said supporting substrate is formed in a convex shape having one or more steps, a plurality of wiring patterns are arranged from a surface of a top step through a surface of a bottom step in the one surface of the supporting substrate,
said probes each electrically connected with one end of each of said wiring patterns are arranged on the surface of the top step while a plurality of first through holes are formed in the bottom step in the one surface of the supporting substrate, and
each of said bumps is electrically connected with the other end of each of said wiring patterns via a conductive material arranged within each of the first through holes.

3. The probe card according to claim 1 or 2, wherein said bumps or said first through holes are arranged at a larger pitch spacing than said probes.

4. The probe card according to claim 1 or 2, wherein second through holes penetrate from the surface of the top step of said one surface of the supporting substrate through the other surface of the supporting substrate.

5. The probe card according to claim 3, wherein the other surface of the supporting substrate is formed with a concave portion therethrough said second through holes penetrate.

6. The probe card according to claims 1, 2 and 3, wherein said one surface of the supporting substrate is formed in a pyramid shape having one or more steps.
